# EUROPEAN PATENT APPLICATION

(11) **EP 0 574 878 A2**
(43) Date of publication of application: **22.12.1993**
(21) Application number: 93109531.9
(22) Date of filing: 15.06.1993
(51) Int. Cl.: C12N 15/34, C12N 15/40

(54) **Barley mild mosaic virus genes**

(30) Priority: 16.06.1992 JP 180624/92
(71) Applicant: DIRECTOR OF NATIONAL AGRICULTURE RESEARCH CENTER, MINSTERY OF AGRICULTURE, FORESTRY AND FISHERIES, Tsukuba-shi, Ibaraki-ken (JP); SAPPORO BREWERIES LIMITED, Tokyo (JP)
(72) Inventor: Kashiwazaki, Satoshi, Tsukuba-shi, Ibaraki-ken (JP); Hibino, Hiroyuki, Tsukuba-shi, Ibaraki-ken (JP); Kuroda, Hisao, c/o Sapporo Breweries Ltd., Nitta-cho, Nitta-gun, Gunma-ken (JP); Ito, Kazutoshi, c/o Sapporo Breweries Ltd., Nitta-cho, Nitta-gun, Gunma-ken (JP)
(74) Representative: VOSSIUS & PARTNER

(57) **Abstract**

DNA containing base sequences which code for the isolated coat protein genes of barley mild mosaic virus.

The determination of the genetic structures of BaMMV coat proteins according to the present invention contribute to the diagnosis of the virus which causes barley yellow mosaic disease, as well as to the production of yellow mosaic disease-resistant barley utilizing the BaMMV coat protein genes.

## Description

The present invention relates to a coat protein gene of barley mild mosaic virus (hereunder also abbreviated to BaMMV).

In the past, yellow mosaic disease, which is a serious blight occurring to barley, has been thought to be caused by barley yellow mosaic virus (hereunder also abbreviated to BaYMV) alone. However, the present inventors and researchers in Germany have recently discovered in yellow mosaic disease-stricken leaves BaMMV, a virus which differs both biochemically and physicochemically from BaYMV, and which also exhibits no immunological cross-reactivity (Kashiwazaki et al., Annals of the Phytopathological Society of Japan, Vol. 55, No.1, 16-25, 1989; Huth, W. et al., Intervirology, Vol. 31, 38-42, 1990).

As mentioned above, BaMMV is thought to have an important connection with yellow mosaic disease, a serious blight of barley, but the details were still unclear regarding its connection with BaYMV and the role both of these play in yellow mosaic disease which occurs in nature. Therefore, determination of the genetic structure of BaMMV is expected to provide an important contribution to the understanding of yellow mosaic disease and to the efficient diagnosis of BaMMV-induced yellow mosaic disease, as well as to the development of methods for the breeding of yellow mosaic disease-resistant barley.

The present inventors, as a result of varied research regarding BaMMV, and particularly the coat protein genes of BaMMV, succeeded in isolating the BaMMV genes, and further identified the BaMMV coat protein genes and discovered that an efficient diagnosis of BaMMV-induced yellow mosaic disease is possible, thus completing the present invention. That is, the present invention relates to DNA containing a base sequence which codes for an isolated coat protein gene of BaMMV.

Fig. 1 is a restriction enzyme cleavage map common to the isolated coat protein genes of barley mild mosaic viruses.

Fig. 2 is a restriction enzyme cleavage map of the isolated coat protein gene of Na1 strain of barley mild mosaic virus.

Fig. 3 a restriction enzyme cleavage map of the isolated coat protein gene of Ka1 strain of barley mild mosaic virus.

The DNA according to the present invention which contains a base sequence which codes for a coat protein gene of BaMMV may be obtained following the method described below.

### (Method of isolating BaMMV)

The isolation of BaMMV may be effected in the same manner as for other plant viruses, by concentration by differential centrifugation, and purification using sucrose density-gradient or caesium chloride equilibrium density-gradient centrifugation, etc.

A common method is one wherein leaves were ground in a buffer solution, for example a 0.1 M citrate buffer solution, a 0.1 M phosphate buffer solution, etc., after which they were filtered with gauze to obtain a crude sap, as in the method of Usugi & Saito.(Annals of the Phytopathological Society of Japan, Vol. 42, No. 1, 1976). Then, after clarification of the sap using an organic solvent such as carbon tetrachloride or ethyl ether, high speed centrifugation (5,000-10,000 x g) and ultracentrifugation (80,000-100,000 x g) may be repeated for separation of the virus from the contaminant to obtain the concentrated virus sample. Further, a purified virus sample may be obtained by subjecting the concentrated virus sample to sucrose density-gradient centrifugation or caesium chloride density-gradient centrifugation, followed by removal and desalting, etc. of the viral layer.

BaMMV strains Na1 and Ka1 were isolated from yellow mosaic disease-stricken barley leaves in the manner described above (Kashiwazaki S. et al., Proceedings of the First Symposium of the International Working Group on Plant Viruses with Fungal Vectors, Braunschweig 105-108, 1990).

### (Separation and identification of BaMMV strains Na1 and Ka1)

Na1 strain and Ka1 strain were collected from yellow mosaic disease-stricken barley leaves grown in the Natajima region of Yamaguchi Prefecture and in Kagawa Prefecture, respectively, and both were subcultured by inoculating to the sap, and then purified. Both strains were preserved at the Virus Disease Control Laboratory of the Department of Plant Protection, Natural Agricaltural Research Center of the Ministry of Agriculture, Forestry and Fisheries.

Antibodies were produced using each of the purified viruses, and upon cross-reaction with purified BaYMV or BaMMV they reacted only with BaMMV, indicating that the separated and purified Na1 and Ka1 strains were of BaMMV (Kashiwazaki S. et al., Proceedings of the First Symposium of the International Working Group on Plant Viruses with Fungal Vectors, Braunschweig, 105-108, 1990).

### (Extraction of RNA from BaMMV)

The method for the extraction of RNA from BaMMV may be any publicly known method, and examples thereof include the guanidine method, hot-phenol method, SDS-phenol method, etc. The extraction of RNA from BaMMV may be achieved by any of these methods.

### (Synthesis of cDNA from RNA)

cDNA may be prepared by a conventional method from the BaMMV RNA extracted in the manner described above. For example, the cDNA may be prepared according to the method reported by Okayama and Berg, the method reported by Shovell, the method reported by Gubler and Hoffman, the S1 nuclease method, the method reported by Rand, etc.

### (Cloning of cDNA)

The cDNA synthesized in the manner described above may be incorporated into a plasmid vector or λ-phage vector either alone or using an adapter or by linker ligation.

### (Determination of base sequence)

The base sequence may be determined according to the Maxam-Gilbert method or the dideoxy method, using the plasmid with which the above obtained cDNA was cloned.

Following the methods described above, the DNA containing the gene which codes for BaMMV coat protein was determined to be that listed in Sequence No. 1 or 2 of the sequence list.

### (Isolation of BaMMV coat protein)

The component proteins of BaMMV particles include genome-binding protein, coat protein, etc. The coat protein may be purified using SDS-polyacrylamide gel electrophoresis (PAGE) or high performance liquid chromatography (HPLC).

### (Determination of the N-terminal amino acid sequence of BaMMV coat protein)

Methods for the determination of N-terminal amino acid sequences of proteins include an enzyme method wherein the amino acids were successively excised from the N-terminus using peptidase to determine the amino acid sequence, and a method wherein the N-terminal amino acids were labelled, and then the labelled amino acids were identified to determine the sequence. Presently, the most useful method is the PITC method, one of the latter type, and an apparatus for automatically determining amino acid sequences is commercially available from Applied Biosystems, Inc. (ABI).

The N-terminal amino acid sequences of the coat proteins of the present viruses were determined by using SDS-PAGE to separate the coat proteins, and then using a protein sequencer manufactured by the above mentioned ABI.

By a comparative examination of the N-terminal amino acid sequences of the BaMMV coat protein determined in the manner described above and the base sequences of the BaMMV genes described below and listed as Sequence Nos. 1 and 2 of the sequence list, the primary structures (amino acid sequences) of the coat proteins were confirmed.

By synthesis of the sequences complementary to the BaMMV coat protein genes determined in the above manner using a chemical synthesis method or a gene manipulation method and hybridization under the conditions described below, an identification is possible which is different from the conventional immunological methods, and the BaMMV coat protein genes isolated under these hybridization conditions were also embodiments of the DNA according to the present invention.

According to the hybridization conditions stipulated in the present specification, hybridization is conducted with 6 x SSC in a 1% SDS solution at 65°C, followed by washing with 1 x SSC in a 0.1% SDS solution at 65°C, and then formation of the hybrid.

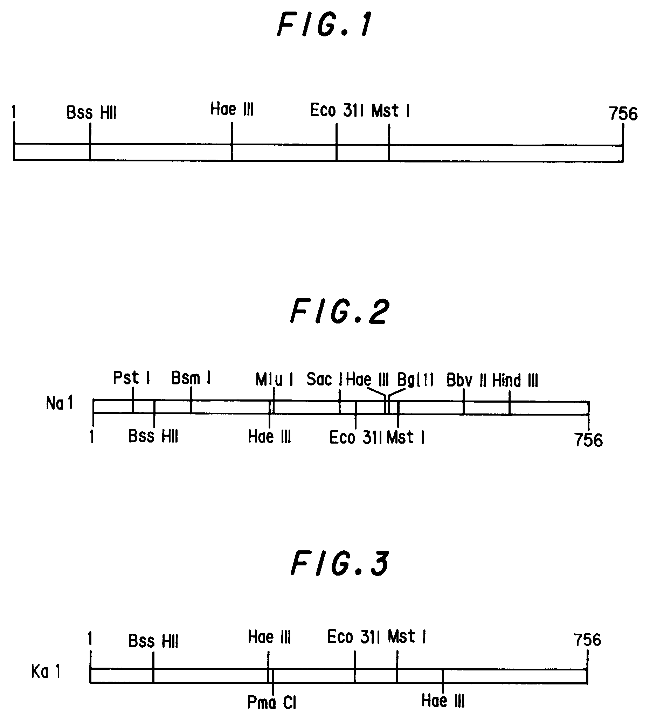

Furthermore, the DNA according to the present invention which is the coat protein gene of the obtained BaMMV strain Na1 or Ka1 has a molecular weight of 480,000 ± 10,000 daltons, and the restriction enzyme cleavage sites shown in Figs. 2 and 3 were discovered to have common sites shown in Fig. 1, which may be mentioned as a characteristic of an embodiment of the DNA according to the present invention.

In addition, by synthesis and analysis of DNA containing the genetic information of BaMMV strain Na1 or Ka1, a gene diagnosis of BaMMV is possible.

### (Diagnosis of BaMMV)

The coat protein genes may be used for the diagnosis of BaMMV A sequence of roughly 20 residues from the base sequence at the N- and C-termini of the coat protein gene of BaMMV strain Na1 or Ka1 is synthesized as the primer DNA. The synthesized primer is used for amplification and detection of the coat protein gene according to the PCR method. The virus may then be distinguished and identified according to the specificity of the primer and by preparing a restriction enzyme cleavage map of the synthesized coat protein gene fragments.

As a concrete illustration, yellow mosaic disease-stricken barley leaves were cut into pieces of approximately 1 cm² and transferred to an ice-cooled Eppendorf tube (TM). To this is added 300 µl of a 0.1 M citrate buffer solution (pH 6.8), and the pieces were ground in an ice-bath using a homogenizer. The ground leaves were then centrifuged at 4°C (5,000 x g, 10 minutes), and 100 µl of the supernatant is transferred to a separate tube. Next, 200 µl of a 30 mM Tris buffer solution (pH 8) containing 3 mM EDT, 0.6% SDS and 600 µg/ml of Protease K (product of Berringer Co.) is added thereto, and the mixture is incubated at 37°C for 30 minutes. It is then cooled on ice, after which extraction is made with phenol, 1 µl of 10 mg/ml glycogen is added thereto, and the RNA is precipitated using ethanol. The excess ethanol is removed by vacuum drying, and the RNA is dissolved in a 10 mM Tris-HCl buffer solution (pH 7.5) containing 1 mM EDTA and kept at -80°C.

Next, a Gene AmpR RNA PCR kit (Perkin Elmer Cetus Instruments, Inc.) is used to amplify and detect the coat protein gene fragments according to the PCR method. The PCR primers used for the detection of Na1 strain were 5'-TCAGGAAAAGATGATCCAGA-3' (20 bases at the N-terminus of the coat protein gene of Na1 strain: SEQ ID No.3) and 5'-CAGTGCTCCGCGAAGAGGCC-3' (20 bases complementary to the C-terminus of the coat protein gene of Na1 strain: SEQ ID No.4), and those used for the detection of Ka1 strain were 5'-GCAGGACACGAAGAACCAGA-3' (20 bases at the N-terminus of the coat protein gene of Ka1 strain: SEQ ID No.5) and 5'-CAGTGCTCCGCGAGGAGGCC-3' (20 bases complementary to the C-terminus of the coat protein of Ka1 strain: SEQ ID No.4). After the PCR reaction, a specific DNA fragment of 753 base pairs is detected only when a primer corresponding to the existing virus is used.

If a more detailed investigation is required, a restriction enzyme cleavage map of the detected specific DNA fragment may be prepared, and then compared with the restriction enzyme cleavage maps shown in Figs. 2 and 3 obtained according to the present invention, for a more precise identification of the virus.

### Examples

A more concrete explanation of the present invention is provided below with reference to the Examples, to which, however, the present invention is not limited.

### Example 1: Purification of BaMMV strain Na1 and BaMMV strain Ka1

One hundred grams of BaMMV-infected leaves were placed in an ice-cooled mortar, 270 ml of a 0.1 M citrate buffer solution (pH 6.8) which had been cooled on ice was added thereto, and the mixture was adequately ground. Unless otherwise specified, the entire procedure was carried out at 4°C. The procedure was repeated 3 times, treating 300 g of infected leaves. The ground solution was filtered with gauze, carbon tetrachloride and ether were added at 1/5 volume each, and the solution was stirred well. Centrifugation was then effected for 10 minutes under conditions of 1000 x g, 4°C to obtain a supernatant. The supernatant was then centrifuged for 15 minutes under conditions of 5000 x g, 4°C to obtain a supernatant which was then centrifuged for 1 hour under conditions of 80,000 x g, 4°C to obtain a precipitate.

Next, for removal of the contaminant, the precipitate was resuspended in a 0.1 M citrate buffer solution and alternately subjected to centrifugation at 5000 x g for 10 minutes and 100,000 x g for 1 hour, which was repeated 3 times to obtain viral particles. For further purification, the particles were suspended in 4 ml of a 0.1 M citrate buffer solution containing 1.33 g of CsCl which was centrifuged at 150,000 x g for 16 hours and then the viral layer was recovered with a syringe. A 0.1 M citrate buffer solution was then added to the viral layer, centrifugation was effected at 55,000 x g for 1 hour to obtain the viral particles as a precipitate, which was then resuspended in a 0.1 M citrate buffer solution and kept as the purified virus.

### Example 2: Extraction of RNA from BaMMV strain Na1 and BaMMV strain Ka1, and purification thereof

The method used for extraction of the RNA from the purified BaMMV strain Na1 or BaMMV strain Ka1 included the SDS-phenol treatment, etc. Of the suspension of BaMMV Na1 purified in Example 1, 0.8 ml (O.D.₂₅₀=4.1) was subjected to centrifugation at 500,000 x g for 20 minutes, and the virus was recovered as a precipitate. This was suspended in 500 µl of a TE buffer solution (10 mM Tris-HCl, 1 mM EDTA, pH 8.0), after which 10 µl of 20% SDS and 20 µl of Protease K (product of Boehringer Mannheim Gmbh, 20 mg/ml aqueous solution) were added thereto and mixed therewith, and the mixture was incubated at 37 °C for 20 minutes.

Next, extraction with phenol was effected twice using a mixed solution of phenol:chloroform (1:1). The aqueous layer was extracted twice with chloroform and 3 times with ether, after which 40 µl of 3 M sodium acetate (pH 4.8), 2 µl of glycogen (product of Boehringer Mannheim Gmbh, 10 mg/ml aqueous solution) and 1 ml of ethanol were added thereto and mixed therewith, and the mixture was allowed to stand at -80°C for 20 minutes. It was then subjected to centrifugation at 15,000 x g for 5 minutes to obtain a precipitate. The obtained precipitate was then washed twice centrifugally using 70% ethanol. The ethanol was removed by drying, after which the precipitate was dissolved in 20 µl of water and kept as the RNA fraction.

### Example 3: Synthesis of BaMMV Na1 cDNA and BaMMV Ka1 cDNA

A cDNA synthesis kit (Amersham Co., cDNA synthesis system plus) was used to synthesize cDNA from the separated and purified RNA. To 6 µl of the RNA solution (0.9 µg/µl TE buffer solution) was added 16.5 µl of autoclaved distilled water, and the mixture was incubated at 65°C for 2 minutes, and then cooled on ice. Next, the cDNA was synthesized following the protocol of Amersham Co. In order to remove the protein and low molecular weight contaminant from the finally obtained cDNA solution, it was passed through a mini-column which had been filled with Sepharose 2B to obtain the object pure cDNA fraction.

### Example 4: Cloning of BaMMV Na1 and BaMMV Ka1 cDNA in plasmid pBluescriptII

(1) For the cloning cDNA of BaMMV Na1 or BaMMV Ka1 in a plasmid, ligation with an adapter was followed by size fractionation. The purified cDNA fraction obtained in Example 3 was precipitated with ethanol, the cDNA was dried, and 5 µl of distilled water was added thereto for dissolution. To this was added 1 µl of EcoRI-NotI-BamHI adapter (10 pmol/µl, product of Takara Shuzo Co.,Ltd.) and 48 µl of A solution and 6 µl of B solution from a DNA ligation kit (product of Takara Shuzo Co.,Ltd.), and the ligation reaction was conducted at 16°C for 2 hours. The reaction solution was subjected to a precipitation process with ethanol and dried, after which the precipitate was dissolved in 5 µl of a TE buffer solution and subjected to electrophoresis on low melting point agarose (SeaKemK^{K} GTG Agarose, product of FMC Co.), and a portion of gel containing 2 kb or more of the cDNA was cut out and transferred to an Eppendorf tube. To this was added 100 µl of a TE buffer solution, and the mixture was incubated at 65°C for 5 minutes to melt the gel, after which it was treated with phenol, precipitated with ethanol, and 10 µl of distilled water was added thereto for dissolution.
(2) Next, in the case of BaMMV Na1, 5 µl of 10 mM ATP, 5 µl of a 10 x phosphorylated buffer solution (660 mM Tris-HCl buffer solution (pH 7.6), 66 mM MgCl₂, 100 mM dithiothreitol, 1 mM spermidine), 1 µl of T₄ polynucleotide kinase and 34 µl of distilled water were added to 5 µl of the sample obtained in (1) above, and the mixture was incubated at 37°C for 1 hour to conduct the phosphorylation reaction. The reaction solution was treated with phenol and precipitated with ethanol, and then dissolved in 5 µl of distilled water.
   Next, after the sample was digested with EcoRI, 1 µl of 0.02 µg/µl plasmid pBluescript SK(+) (product of Stratagene Co.) which had been subjected to dephosphorylation, and 48 µl of A solution and 6 µl of B solution of the DNA ligation kit (product of Takara Shuzo Co. ,Ltd.) were added thereto, and the ligation reaction was conducted at 16°C for 2 hours. In the case of Ka1, the sample obtained in (1) above was digested with SmaI, and 1 µl of 0.02 µg/µl plasmid pBluescript SK(+) which had been subjected to dephosphorylation, and 48 µl of A solution and 6 µl of B solution of the DNA ligation kit (product of Takara Shuzo Co., Ltd.)were added thereto, and the ligation reaction was conducted at 16°C for 2 hours.
(3) Competent cells of E. coli NM522 (obtained from Stratagene Co.) were prepared, and the reaction solution obtained in (2) above was mixed with 200 µl of the competent cells on ice, and then allowed to stand for 30 minutes. Next, the mixture was incubated at 42°C for 2 minutes, and then immediately transferred to ice, upon which 0.9 ml of SOC medium was added thereto and incubation was effected at 37°C. After 1 hour, the mixture was plated out onto 10 LB agar plates (9 cm diameter) each containing 150 µg/ml of 0.02% 5-bromo-4-chloro-3-indolyl-β-D-galactopyranoside and 0.5 mM of isopropylthiogalactoside, and cultured at 37°C overnight. The plasmid DNA was extracted from E.coli taken from the white colony, and E.coli possessing plasmids which included long cDNA fragments were selected, and kept.
(4) Next, the following experiment was carried out to obtain cDNA derived from RNA1. As indicated in Example 2, the RNA of BaMMV Na1 or BaMMV Ka1 was purified and subjected to electrophoresis on low melting point agarose (SeaKemK GTG Agarose, product of FMC Co.), and a portion of gel containing the RNA1 band was cut out and transferred to an Eppendorf tube. To this was added 100 µl of a TE buffer solution, and the mixture was incubated at 65°C for 5 minutes to melt the gel, after which it was treated with phenol, precipitated with ethanol, dried, and then dissolved in 10 µl of distilled water. Using this RNA solution, dot hybridization was effected using an ECL system (product of Amersham Co.) with the cloned cDNA obtained in (3) above as the probe. As a result, four E. coli strains possessing plasmids which included the cDNA fragments derived from RNA1: Na1SK2, Na1SK15, Ka1SK11 and Ka1SK46 were obtained. The plasmids in each of the strains had cloned cDNA fragments of 3.5Kb, 3.5Kb, 4Kb and 3.8Kb, respectively.

### Example 5: Determination of the base sequences of cDNA derived from genomic RNA of BaMMV strains Na1 and Ka1

The overnight-cultured solution of E.coli NM522 including plasmids Na1SK2(FERM BP-4325), Na1SK15, Ka1SK11(FERM BP-4326) and Ka1SK46 was inoculated into 3 ml of 2 YT culture mediums (16 g/l Bactotrypton, 10 g/l yeast extract, 10 g/l NaCl, pH 7.2), and culturing was effected at 37°C. After inoculation, helper phage R408 was added to a m.o.i. of 20, and was further cultured overnight. The phage was separated by a conventional method, and the single-stranded DNA was purified and then used as the template for the determination of the base sequence according to the dideoxy method using a 373A DNA sequencer manufactured by ABI Co.

### Example 6: Determination of the N-terminal amino acid sequence of the coat protein

Using 10 µl of a solution (1.5 mg/ml, 0.1 M citrate buffer solution, pH 6.8) which contained BaMMV strain Na1 or Ka1, the coat protein was separated by SDS-PAGE, after which it was transfered onto a ProBlott (tradename) membrane manufactured by ABI Co. according to the Western blotting method, and dyed with Coomassie blue. After dyeing, the band corresponding to the coat protein was cut out, and the N-terminal amino acid sequence was determined using an ABI477A protein sequencer manufactured by ABI Co.

The results clearly showed that the N-terminal amino acid sequence of the coat protein was SGKDDPDPIV (SEQ ID No. 6) for strain Na1 and AGHEEPDPIV (SEQ ID No. 7) for strain Ka1. From the results in Example 5 and the above results of the N-terminal amino acid sequence of the BaMMV coat protein, the primary structure of the coat protein was revealed.

The coat protein of BaMMV strain Na1 was shown to consist of amino acids 798 to 1048 of the amino acid sequence listed as Sequence No. 1 in the sequence list. The base sequence was shown to correspond to the region of bases 2392 to 3144 of the base sequence listed as Sequence No. 1 in the sequence list.

The coat protein of BaMMV strain Ka1 was shown to consist of amino acids 952 to 1202 of the amino acid sequence listed as Sequence No.1 in the sequence list. The base sequence was shown to correspond to the region of bases 2854 to 3606 of the base sequence listed as Sequence No. 1 in the sequence list.

As mentioned above, the determination of the genetic structures of BaMMV coat proteins according to the present invention may contribute to the diagnosis of the virus which causes barley yellow mosaic disease, as well as to the production of yellow mosaic disease-resistant barley utilizing the BaMMV coat protein genes.

"Sequence Listing"

SEQ ID NO:1

SEQUENCE TYPE:Nucleotide with corresponding protein

SEQUENCE LENGTH:3485 base pairs

PROPERTIES:

TTT GAA CTC GAC ATG GAA CAA CAC GAT CCT GAG ACT ATC GCC AAA 45

Phe Glu Leu Asp Met Glu Gln His Asp Pro Glu Thr Ile Ala Lys

                5                   10                  15

CTT GGA GGC GAA GTT GGT TTC AGG ATG AAC AGG TAT GAT CTA CGC 90

Leu Gly Gly Glu Val Gly Phe Arg Met Asn Arg Tyr Asp Leu Arg

                20                  25                  30

CAA GTT GGT GCC ACA CGA TAC ATC AAC CCA AAA GCA CAA ACA TCC 135

Gln Val Gly Ala Thr Arg Tyr Ile Asn Pro Lys Ala Gln Thr Ser

                35                  40                  45

GCA GCA ACG CTT GAA GGC ATG ACC ATG AAA CCA ATG AGT GCT TTC 180

Ala Ala Thr Leu Glu Gly Met Thr Met Lys Pro Met Ser Ala Phe

                50                  55                  60

ACC ATT GAC AGC GCG AAG ATG GTC GGT TTT ATT AAA ACT GCA AAA 225

Thr Ile Asp Ser Ala Lys Met Val Gly Phe Ile Lys Thr Ala Lys

                65                  70                  75

GAC ACA CTC AAC TGC ATT CTA TAT GGC GAC TGG ATC ATA GCT CCC 270

Asp Thr Leu Asn Cys Ile Leu Tyr Gly Asp Trp Ile Ile Ala Pro

                80                  85                  90

GCA CAC ATA CAG CAG GGC GAA GGG GAC ATA ACA TTC ATC TTC CAG 315

Ala His Ile Gln Gln Gly Glu Gly Asp Ile Thr Phe Ile Phe Gln

                95                  100                 105

CAC ACA CAG TTT ACA ACT ACC ACC GAG CGT TTA GCA AGC TAC GGG 360

His Thr Gln Phe Thr Thr Thr Thr Glu Arg Leu Ala Ser Tyr Gly

                110                 115                 120
ATT CGA CAG TTC AAA GGC CTT GAC CTT GTT GTC ATT CGA CGC CCC 405
Ile Arg Gln Phe Lys Gly Leu Asp Leu Val Val Ile Arg Arg Pro
                125                 130                 135
CAG CAG ATA CGC GCA GTT AAG AAG GAC ATG CGC GCC AGC ATT CTT 450
Gln Gln Ile Arg Ala Val Lys Lys Asp Met Arg Ala Ser Ile Leu
                140                 145                 150
GAC ACA CCC ACT GAA GTG CAA ATG CTC TAC CTA TCA ACC AAA GGC 495
Asp Thr Pro Thr Glu Val Gln Met Leu Tyr Leu Ser Thr Lys Gly
                155                 160                 165
GGC AAG TAT CAA GTG TCC ACG TCT GCT GTG TGT TTC CCA CAC TAT 540
Gly Lys Tyr Gln Val Ser Thr Ser Ala Val Cys Phe Pro His Tyr
                170                 175                 180
AAC AAC CGA TGG GGA CAC GTC ATT TCA ACA GCC GAA GGC ATG TGC 585
Asn Asn Arg Trp Gly His Val Ile Ser Thr Ala Glu Gly Met Cys
                185                 190                 195
GGC TGC ATT GTT TTC AAC CCC ACC ACA AAC CAC ATT GTT GGG ATT 630
Gly Cys Ile Val Phe Asn Pro Thr Thr Asn His Ile Val Gly Ile
                200                 205                 210
CAC GTC TCC TAC AAT GAC ACG CGC AGA AGA AAC GAG TTT CAA GCT 675
His Val Ser Tyr Asn Asp Thr Arg Arg Arg Asn Glu Phe Gln Ala
                215                 220                 225
TTT ACA AGT GAC GTT CTA ACA ACA ATC AAC GCA CCA GGA CAC GAA 720
Phe Thr Ser Asp Val Leu Thr Thr Ile Asn Ala Pro Gly His Glu
                230                 235                 240
ATA CCC TTC AGT CCA TGG GTT TTT GAC TGG AAG TTT TGC GGG TAC 765
Ile Pro Phe Ser Pro Trp Val Phe Asp Trp Lys Phe Cys Gly Tyr
                245                 250                 255
ACA ACG AAA CCA CGG AAC ATG CAA AGT GCA CCA AGC ACG CTT GAA 810

Thr Thr Lys Pro Arg Asn Met Gln Ser Ala Pro Ser Thr Leu Glu
                260                 265                 270
CGA CTC AAC ATC AAC GCC ACA GGT TTT GGT TTC AAA CTC AAT GCA 855
Arg Leu Asn Ile Asn Ala Thr Gly Phe Gly Phe Lys Leu Asn Ala
                275                 280                 285
CAG GGG ATC AAA CCT GCC ATG CTT CGC TCC ACA GAA ACA TTC TCC 900
Gln Gly Ile Lys Pro Ala Met Leu Arg Ser Thr Glu Thr Phe Ser
                290                 295                 300
AGG GAG TTC CCG AAC ACA CAG TTC AAG CTC ATC GGA GAA GTG AAG 945
Arg Glu Phe Pro Asn Thr Gln Phe Lys Leu Ile Gly Glu Val Lys
                305                 310                 315
AAG GGA CTC ATT GAT AAA CAC ACG ATC ACA GGA GAG AAC CCC TAT 990
Lys Gly Leu Ile Asp Lys His Thr Ile Thr Gly Glu Asn Pro Tyr
                320                 325                 330
TTC CTT GAA TTC CTC AAC ACA TTC AAA CCA TAC CAA TGG GTA CAA 1035
Phe Leu Glu Phe Leu Asn Thr Phe Lys Pro Tyr Gln Trp Val Gln
                335                 340                 345
GCT TTC ATG GAC GAG TAC GCA CCC TCC ATC CTT GCC TAC GAC GCG 1080
Ala Phe Met Asp Glu Tyr Ala Pro Ser Ile Leu Ala Tyr Asp Ala
                350                 355                 360
TAC TTC AAG GAC TTG AAG AAG TAC GAT AGG CCG CCA CAC GCA AGT 1125
Tyr Phe Lys Asp Leu Lys Lys Tyr Asp Arg Pro Pro His Ala Ser
                365                 370                 375
GTG TTC TGT GAG GAC ACA TTG ACC AAA GCC AAA CAC AAA ATG ATT 1170
Val Phe Cys Glu Asp Thr Leu Thr Lys Ala Lys His Lys Met Ile
                380                 385                 390
AAG ATT CTC GAA GAG GCA GGT ATG GGC AGA ACC CTA GTA CGA ACC 1215
Lys Ile Leu Glu Glu Ala Gly Met Gly Arg Thr Leu Val Arg Thr
                395                 400                 405

ACC GAG CAA GTC TTA CTT GAC ATG GCC TGG ACC ACC TCT GGT GGA 1260
Thr Glu Gln Val Leu Leu Asp Met Ala Trp Thr Thr Ser Gly Gly
                410                 415                 420
CCC TTG TAC CAT GGC AAG AAG ATT GAC ATT GTA CAA CAC TTA TCT 1305
Pro Leu Tyr His Gly Lys Lys Ile Asp Ile Val Gln His Leu Ser
                425                 430                 435
GAT GAT GAG CTT GTG CAA TTC AGT GAA GCC TGC CAA CAG GCT CTC 1350
Asp Asp Glu Leu Val Gln Phe Ser Glu Ala Cys Gln Gln Ala Leu
                440                 445                 450
ATC ACT GGA ACG CTA GAT GGA GTC TGG AAC GGA TCA CTC AAA GCC 1395
Ile Thr Gly Thr Leu Asp Gly Val Trp Asn Gly Ser Leu Lys Ala
                455                 460                 465
GAG CTC CGC TCT TCG CAG AAG ATC CTC GAG CGG AAA ACT CGT GTC 1440
Glu Leu Arg Ser Ser Gln Lys Ile Leu Glu Arg Lys Thr Arg Val
                470                 475                 480
TTC ACT GCT GCC CCC ATA ACA AGT CTA ATT GCG ATG AAA TAC TAC 1485
Phe Thr Ala Ala Pro Ile Thr Ser Leu Ile Ala Met Lys Tyr Tyr
                485                 490                 495
GTG GAC GAT TTC AAC AAA CAA TTC TAC AAG ACT CAT CTC AAA GCA 1530
Val Asp Asp Phe Asn Lys Gln Phe Tyr Lys Thr His Leu Lys Ala
                500                 505                 510
CCA CAT ACA GTT GGT ATC AAC AAG TTT AAC CGC GGA TGG CAG AAT 1575
Pro His Thr Val Gly Ile Asn Lys Phe Asn Arg Gly Trp Gln Asn
                515                 520                 525
CTC TAT GAG AAG CTC AAC AAA CCT GGA TGG ACG CAT GGG AGT GGG 1620
Leu Tyr Glu Lys Leu Asn Lys Pro Gly Trp Thr His Gly Ser Gly
                530                 535                 540
GAT GGC TCC AGG TTT GAT AGC TCG ATT GAT GGA TTC CTC TTC GAT 1665
Asp Gly Ser Arg Phe Asp Ser Ser Ile Asp Gly Phe Leu Phe Asp

                545                 550                 555
GTC ATC AAA GAC ATC CGA AAG CAC TTC ATG GAT GCA GAA CAC CAC 1710
Val Ile Lys Asp Ile Arg Lys His Phe Met Asp Ala Glu His His
                560                 565                 570
AAG CAG CTT GAC ACA ATC TAT GAA GAA ATT GTT AAC ACG AAG ATA 1755
Lys Gln Leu Asp Thr Ile Tyr Glu Glu Ile Val Asn Thr Lys Ile
                575                 580                 585
TGT TTA GCA AAT GGT CTA GTC ATC CAA AAG AAT TGT GGA AAC AAT 1800
Cys Leu Ala Asn Gly Leu Val Ile Gln Lys Asn Cys Gly Asn Asn
                590                 595                 600
AGC GGA CAG CCG AGC ACT GTC GTC GAC AAC ACA CTC GCA CTC ATG 1845
Ser Gly Gln Pro Ser Thr Val Val Asp Asn Thr Leu Ala Leu Met
                605                 610                 615
ACT TCT TTT CTT TAT GCA TAC GCC CGA CTC ACT GGA GAT GAC ACC 1890
Thr Ser Phe Leu Tyr Ala Tyr Ala Arg Leu Thr Gly Asp Asp Thr
                620                 625                 630
TTT GAA CTC ATG GAC GAA AAC TTC GTT TTC GTC TGC AAC GGC GAC 1935
Phe Glu Leu Met Asp Glu Asn Phe Val Phe Val Cys Asn Gly Asp
                635                 640                 645
GAC AAC AAG TTC GCC ATG TCA CCC AGC TTC ATG GTG AAG TTC GGG 1980
Asp Asn Lys Phe Ala Met Ser Pro Ser Phe Met Val Lys Phe Gly
                650                 655                 660
TGC GAT TTC TCC CCA TTC CTT TCT GAG CTA GGG CTA ACC TAC GAG 2025
Cys Asp Phe Ser Pro Phe Leu Ser Glu Leu Gly Leu Thr Tyr Glu
                665                 670                 675
TTC GAC GAA GCC ACC GAA GAC ATC TGT GAA AAC CCC TAC ATG TCC 2070
Phe Asp Glu Ala Thr Glu Asp Ile Cys Glu Asn Pro Tyr Met Ser
                680                 685                 690
TTG ACC ATG GTT CGC ACC TCG TTC GGG ATC GGC TTC TCT CTC TCC 2115

Leu Thr Met Val Arg Thr Ser Phe Gly Ile Gly Phe Ser Leu Ser
                695                 700                 705
ATC GAA CGC ATC GTT GCA ATC CTG CAA TGG AGC CGT GCT GGT GGA 2160
Ile Glu Arg Ile Val Ala Ile Leu Gln Trp Ser Arg Ala Gly Gly
                710                 715                 720
GTC CTG CAT GCC TAT CTC TCT GGC ATT GCT GCT CTT TTC GAG TCC 2205
Val Leu His Ala Tyr Leu Ser Gly Ile Ala Ala Leu Phe Glu Ser
                725                 730                 735
TTC AAC ACA CCC AAA CTC TTC AAC CTC GTG CAC ACA TAC CTC CTC 2250
Phe Asn Thr Pro Lys Leu Phe Asn Leu Val His Thr Tyr Leu Leu
                740                 745                 750
TGG CTA GTC ACG GAG CAT GAG GAG GAA CTT TTT TCG ATG ATG GAA 2295
Trp Leu Val Thr Glu His Glu Glu Glu Leu Phe Ser Met Met Glu
                755                 760                 765
CTC AAG GAT ATG TTC ATG CCT CTG CCC ACT AAG GAA CAG ATA GCC 2340
Leu Lys Asp Met Phe Met Pro Leu Pro Thr Lys Glu Gln Ile Ala
                770                 775                 780
TTG TTG CAC TAC GTA GGC ACT GAA CCC ATC ATG GAG GAA ACA TAT 2385
Leu Leu His Tyr Val Gly Thr Glu Pro Ile Met Glu Glu Thr Tyr
                785                 790                 795
CTG CAA TCA GGA AAA GAT GAT CCA GAT CCC ATT GTT CCT CCA GTT 2430
Leu Gln Ser Gly Lys Asp Asp Pro Asp Pro Ile Val Pro Pro Val
                800                 805                 810
TCT GAC ACT GAT CTC ACA AAC ATG GCT GCA GCA CCA CCC GAC AAC 2475
Ser Asp Thr Asp Leu Thr Asn Met Ala Ala Ala Pro Pro Asp Asn
                815                 820                 825
AGA AGG TCG CGC GCC GTC GTC CCC CGT GGG ACC AGT GAC TGG AAT 2520
Arg Arg Ser Arg Ala Val Val Pro Arg Gly Thr Ser Asp Trp Asn
                830                 835                 840

TTG CCT GAA CCC AAG ATG CGA ATG CTA GGT TTC AAG TCC AAA ATC 2565
Leu Pro Glu Pro Lys Met Arg Met Leu Gly Phe Lys Ser Lys Ile
                845                 850                 855
AAC ATA GAA ACG CTC GCC GAT GTC CCC GAA GGG TAT ATG AAT ACC 2610
Asn Ile Glu Thr Leu Ala Asp Val Pro Glu Gly Tyr Met Asn Thr
                860                 865                 870
TTT GCG TCC GTT GCA ACT GAA ACG CAA CGT AGG AAA TGG GAA GAG 2655
Phe Ala Ser Val Ala Thr Glu Thr Gln Arg Arg Lys Trp Glu Glu
                875                 880                 885
GCC ACG CGT GGT GAT TTT GGC ATC ACC GAC AAC GAG AAG TGG GAA 2700
Ala Thr Arg Gly Asp Phe Gly Ile Thr Asp Asn Glu Lys Trp Glu
                890                 895                 900
AAG CTG CTC ATT GCT GCC TGC ATA TAT TTC GCA GAC AAT GGC ACT 2745
Lys Leu Leu Ile Ala Ala Cys Ile Tyr Phe Ala Asp Asn Gly Thr
                905                 910                 915
TCA CCA AAC TTC GAT GAG GAG CTC ACT ATG GAA GTC AAT GGC GGT 2790
Ser Pro Asn Phe Asp Glu Glu Leu Thr Met Glu Val Asn Gly Gly
                920                 925                 930
CTC AAC TCC ATC AAA GAG TAT CCC GTC CGC CCT TTT GTT GTC AGG 2835
Leu Asn Ser Ile Lys Glu Tyr Pro Val Arg Pro Phe Val Val Arg
                935                 940                 945
GCC AAA AAG ATC TCC ACA CTG CGC AGG ATC TTT CGC TGT TAC TCC 2880
Ala Lys Lys Ile Ser Thr Leu Arg Arg Ile Phe Arg Cys Tyr Ser
                950                 955                 960
ATT GAG ACG AAA CTA ATG TTC GTG AAG CTG AGG CGC GTA CCA CAA 2925
Ile Glu Thr Lys Leu Met Phe Val Lys Leu Arg Arg Val Pro Gln
                965                 970                 975
TGG GCT ATA AAG CAC GGG TGC CTT GAT GAA ATC GTC TTC GAT TTC 2970
Trp Ala Ile Lys His Gly Cys Leu Asp Glu Ile Val Phe Asp Phe

                980                 985                 990
ATG ATC CCA GAC CAG TTC ACT TCC AGA ACT GCA CTC GAA ACA CTG 3015
Met Ile Pro Asp Gln Phe Thr Ser Arg Thr Ala Leu Glu Thr Leu
                995                 1000                1005
AAG CAG ACC AAG CTT GCT GCC ATT GGT GTT GGC ACA AGC AAT TCT 3060
Lys Gln Thr Lys Leu Ala Ala Ile Gly Val Gly Thr Ser Asn Ser
                1010                1015                1020
CTC CTC ACC TCC GAA CAG ACG AAC ATG AGA ACC ACT GAA ACC CGA 3105
Leu Leu Thr Ser Glu Gln Thr Asn Met Arg Thr Thr Glu Thr Arg
                1025                1030                1035
AGG CGC AAC GAC TAC GAC GGT CAC GAG GCG CTT CTC CGG 3144
Arg Arg Asn Asp Tyr Asp Gly His Glu Ala Leu Leu Arg
1040 1045 1048
TAGGTATCTC ACACAGTTCC ATTTCCTTTT CAGCAATGCA TTTAAACACG CTTCCAATTA 3204
CATCAAGCGT CAAGAGTTGT TCCCGCTTGC AGTTAATCCT TATGGGGTGG CACTTTGTGT 3264
TATGCAATGG ACAGTTCAAT GACGCAATCA ACCATCATTG GTTGGTGACA ACGCAGCATG 3324
TTCTTTCTTT TCACATCAAG CGACCTGCAC ATCGCCCGCT TACAGCTAAT CCTACCTGAG 3384
GATGGCACTC TGTGTTATGT AATGGAATGT GCAATCTCGC AACCAACCGT CATTGGTTGA 3444
TGGTGCGGCC AAAACCCGCA CTCCTGTATA CCGGAAAGGT T 3485

SEQ ID NO:2
SEQUENCE TYPE:Nucleotide with corresponding protein
SEQUENCE LENGTH:3946 base pairs

TTA TGG TTG CCG CCT TAC TCA TGT ATC TCG TTT TCT GGG TTT CTC 45
Leu Trp Leu Pro Pro Tyr Ser Cys Ile Ser Phe Ser Gly Phe Leu
                5                   10                  15
CCA GGA AAC AGG ACG ACA TCA CCA TTG AGG GGA AAG GCA GGG CAT 90
Pro Gly Asn Arg Thr Thr Ser Pro Leu Arg Gly Lys Ala Gly His

                20                  25                  30
ACA ACC GTG ACA AGC GCA TGG GGT ACG ACT CCT ACG AGG AAG ACG 135
Thr Thr Val Thr Ser Ala Trp Gly Thr Thr Pro Thr Arg Lys Thr
                35                  40                  45
AAG TTC GCA ACA AGA TCA ACA AAA AGT TCA AAG AGC GCA GAC TTA 180
Lys Phe Ala Thr Arg Ser Thr Lys Ser Ser Lys Ser Ala Asp Leu
                50                  55                  60
AGG TTC TCC AAT AAC TCC AAG CCG GAA ACA AGC TCG AAA TAC CGC 225
Arg Phe Ser Asn Asn Ser Lys Pro Glu Thr Ser Ser Lys Tyr Arg
                65                  70                  75
AAT CTG AAG CAA GAA TTT GTT AAC TTC TAT AAT CTC AAA ACC GAT 270
Asn Leu Lys Thr Asp Ala Asn Val Leu Gln Ala Val Phe Thr Ala
                80                  85                  90
GCG AAC GTG CTA CAA GCC GTG TTC ACT GCA ATG GAC GGG GCC GTC 315
Asn Leu Lys Gln Glu Phe Val Asn Phe Tyr Met Asp Gly Ala Val
                95                  100                 105
CTT CTT CAA ACC GAA AAT CCA ATG GCA GAC ATT GGC CAT GTC AAT 360
Leu Leu Gln Thr Glu Asn Pro Met Ala Asp Ile Gly His Val Asn
                110                 115                 120
AGA CTG CTG CAG GAC CAC TTT GAA GAC AGT GAG AGC CAA GCT GTG 405
Arg Leu Leu Gln Asp His Phe Glu Asp Ser Glu Ser Gln Ala Val
                125                 130                 135
CAT GAG GGC CTC AAC ACC ATG GTC AAG TGC CAC CTA ACT ATG AAA 450
His Glu Gly Leu Asn Thr Met Val Lys Cys His Leu Thr Met Lys
                140                 145                 150
GAT GGA AGA CAG TTT GAG CTC GAC ATG GAA CAA CAC GAT CCC GAA 495
Asp Gly Arg Gln Phe Glu Leu Asp Met Glu Gln His Asp Pro Glu
                155                 160                 165
ACT GTC GCC AAA CTT GGG GGC GAG GTT GGT TTC AGA ATG AAC AGA 540

Thr Val Ala Lys Leu Gly Gly Glu Val Gly Phe Arg Met Asn Arg
                170                 175                 180
GAC GAC TTA CGT CAA GTT GGA GCC ACA CGA TAC ATC AAC CCA AGA 585
Asp Asp Leu Arg Gln Val Gly Ala Thr Arg Tyr Ile Asn Pro Arg
                185                 190                 195
GCA CAA ACA TCT GCA GCG ACG CTC GAA GGC ATG ACC ATG AAA CCC 630
Ala Gln Thr Ser Ala Ala Thr Leu Glu Gly Met Thr Met Lys Pro
                200                 205                 210
ATG AGT TCT TTC ACC ATT GAC AGC GCA AAG ATG GTT GGC TTC ATC 675
Met Ser Ser Phe Thr Ile Asp Ser Ala Lys Met Val Gly Phe Ile
                215                 220                 225
AAG ACC GCA AAA GAC ACA CTC AAC TGC ATC CTT TAC GGC GAC TGG 720
Lys Thr Ala Lys Asp Thr Leu Asn Cys Ile Leu Tyr Gly Asp Trp
                230                 235                 240
ATT ATA GCT CCT GCG CAC ACA CAG CAG GGC GAG GGG GAC ATA ACA 765
Ile Ile Ala Pro Ala His Thr Gln Gln Gly Glu Gly Asp Ile Thr
                245                 250                 255
TTC ATC TTC CAG CAC GTA CAA TTC ACG ACC ACA ACT GAG CGT TTA 810
Phe Ile Phe Gln His Val Gln Phe Thr Thr Thr Thr Glu Arg Leu
                260                 265                 270
GCT AGC TAT GGC ATT CGA CAA TTC AAA GGT CTT GAC CTT GTT GTC 855
Ala Ser Tyr Gly Ile Arg Gln Phe Lys Gly Leu Asp Leu Val Val
                275                 280                 285
ATC CGG CGC CCT CAG CAG ATA CGC GCA GTC AAG AAG GAC ATG CGT 900
Ile Arg Arg Pro Gln Gln Ile Arg Ala Val Lys Lys Asp Met Arg
                290                 295                 300
GCT AGC ATT CTC GAC ACA CCC ACT GAA ATA CAA ATG CTC TAC TTG 945
Ala Ser Ile Leu Asp Thr Pro Thr Glu Ile Gln Met Leu Tyr Leu
                305                 310                 315

TCC ACT AAA GGT GGT AAG TAC CAA GTG TCC ACG TCC GCT GTG TGC 990
Ser Thr Lys Gly Gly Lys Tyr Gln Val Ser Thr Ser Ala Val Cys
                320                 325                 330
TTT CCA CAC TAC AAT AAC CGA TGG GGA CAC GTC ATT TCA ACA GCA 1035
Phe Pro His Tyr Asn Asn Arg Trp Gly His Val Ile Ser Thr Ala
                335                 340                 345
GAA GGC ATG TGC GGC TGT ATT GTA TTC AAC CCC ACC ACG AAC CAC 1080
Glu Gly Met Cys Gly Cys Ile Val Phe Asn Pro Thr Thr Asn His
                350                 355                 360
ATC GTT GGA ATC CAC GTC TCC TAC AAC GAC ACG CGC AGA AGA AAT 1125
Ile Val Gly Ile His Val Ser Tyr Asn Asp Thr Arg Arg Arg Asn
                365                 370                 375
GAG TTT CAA GCT TTC ACA AGT GAC GTT CTG ACA ACG ATT AAT GCA 1170
Glu Phe Gln Ala Phe Thr Ser Asp Val Leu Thr Thr Ile Asn Ala
                380                 385                 390
CCC GGA CAC GAG ATA CCT TTC AGC CCA TGG ATC TTC GAC TGG AAG 1215
Pro Gly His Glu Ile Pro Phe Ser Pro Trp Ile Phe Asp Trp Lys
                395                 400                 405
TTT TGT GGG TAC ACA ACA AAA CCA CGA AAC ATG CAA AGC GCA CCG 1260
Phe Cys Gly Tyr Thr Thr Lys Pro Arg Asn Met Gln Ser Ala Pro
                410                 415                 420
AGC ACA CTC GAG CGA CTC AAC ATC AAT GCC ACA GGA TTT GGT TTC 1305
Ser Thr Leu Glu Arg Leu Asn Ile Asn Ala Thr Gly Phe Gly Phe
                425                 430                 435
AAG CTC GAT GCA CAG GAG ATT AAG CCA GCC ATG CTT CGT TCC ACA 1350
Lys Leu Asp Ala Gln Glu Ile Lys Pro Ala Met Leu Arg Ser Thr
                440                 445                 450
GAG TCG TTC TCC AGG GAG TTT CCA GAC ACA CAG TTT AAA CTC ATT 1395
Glu Ser Phe Ser Arg Glu Phe Pro Asp Thr Gln Phe Lys Leu Ile

                455                 460                 465
GGG GAA GTG AAG AAA GGA CTC ATC GAC AAA CAC ACA ATC ACA GGA 1440
Gly Glu Val Lys Lys Gly Leu Ile Asp Lys His Thr Ile Thr Gly
                470                 475                 480
GAG AAC CCA TAC TTC CTT GAA TTC CTG AAC ACA TTC AAA CCA TAC 1485
Glu Asn Pro Tyr Phe Leu Glu Phe Leu Asn Thr Phe Lys Pro Tyr
                485                 490                 495
CAA TGG GTG CAG GCC TTC ATG GAC GAG TAC GCA CCC TCC ATC CTT 1530
Gln Trp Val Gln Ala Phe Met Asp Glu Tyr Ala Pro Ser Ile Leu
                500                 505                 510
GCC TAT GAC GCG TAT TTC AAA GAT CTG AAG AAA TAT GAT AGG CCA 1575
Ala Tyr Asp Ala Tyr Phe Lys Asp Leu Lys Lys Tyr Asp Arg Pro
                515                 520                 525
CCA CAC GCA AAT GTT TTC TGT CAG GAC ACA CTA ACC AAA GCT AAA 1620
Pro His Ala Asn Val Phe Cys Gln Asp Thr Leu Thr Lys Ala Lys
                530                 535                 540
CAT AAG ATG ATC AGC ATC CTC GAG GAA GCA GGC ATG GGA AGG ACC 1665
His Lys Met Ile Ser Ile Leu Glu Glu Ala Gly Met Gly Arg Thr
                545                 550                 555
CTA GTA CGA ACC ACC GAG CAG GTC TTG CTT GAC ATG GCG TGG ACC 1710
Leu Val Arg Thr Thr Glu Gln Val Leu Leu Asp Met Ala Trp Thr
                560                 565                 570
ACC TCC GGT GGA CCC TTG TAC CAT GGT AAG AAG ATC GAC ATT GTG 1755
Thr Ser Gly Gly Pro Leu Tyr His Gly Lys Lys Ile Asp Ile Val
                575                 580                 585
CAA CAC TTG TCT GAC GAC GAG CTT GTA CAG TTC AGT GAG GCC TGC 1800
Gln His Leu Ser Asp Asp Glu Leu Val Gln Phe Ser Glu Ala Cys
                590                 595                 600
CAA CAG GCT CTT ATC ACT GGA ACG CTG GAT GGA GTC TGG AAC GGA 1845

Gln Gln Ala Leu Ile Thr Gly Thr Leu Asp Gly Val Trp Asn Gly
                605                 610                 615
TCA CTC AAA GCC GAG CTG CGC TCT TCG CAG AAA ATC CTC GAA CGG 1890
Ser Leu Lys Ala Glu Leu Arg Ser Ser Gln Lys Ile Leu Glu Arg
                620                 625                 630
AAA ACC CGC GTT TTT ACT GCT GCA CCC ATA ACA AGC CTC ATT GCG 1935
Lys Thr Arg Val Phe Thr Ala Ala Pro Ile Thr Ser Leu Ile Ala
                635                 640                 645
ATG AAA TAC TAT GTG GAT GAT TTC AAC AAA CAG TTC TAT AAA ACC 1980
Met Lys Tyr Tyr Val Asp Asp Phe Asn Lys Gln Phe Tyr Lys Thr
                650                 655                 660
CAT CTC AAA GCA CCG CAC ACA GTC GGA ATC AAC AAG TTC AAC CGC 2025
His Leu Lys Ala Pro His Thr Val Gly Ile Asn Lys Phe Asn Arg
                665                 670                 675
GGA TGG CAG AAT CTG TAT GAG AAG CTC AAC AAA CCC GGA TGG ACG 2070
Gly Trp Gln Asn Leu Tyr Glu Lys Leu Asn Lys Pro Gly Trp Thr
                680                 685                 690
CAT GGG AGT GGG GAC GGT TCC AGG TTT GAC AGT TCC ATT GAT GGA 2115
His Gly Ser Gly Asp Gly Ser Arg Phe Asp Ser Ser Ile Asp Gly
                695                 700                 705
TTC CTT TTC GAT GTC ATC AAG GAC ATT CGA AAG CAT TTC ATG GAC 2160
Phe Leu Phe Asp Val Ile Lys Asp Ile Arg Lys His Phe Met Asp
                710                 715                 720
ACG GAA CAC CAC AAA GAA CTC GAC ACA ATC TAC GAA GAG ATA GTC 2205
Thr Glu His His Lys Glu Leu Asp Thr Ile Tyr Glu Glu Ile Val
                725                 730                 735
AAC ACG AAG ATA TGT CTG GCG AAC GGC CTG GTC ATT CAG AAG AAT 2250
Asn Thr Lys Ile Cys Leu Ala Asn Gly Leu Val Ile Gln Lys Asn
                740                 745                 750

TGC GGG AAC AAC AGT GGG CAG CCA AGC ACT GTT GTG GAC AAC ACA 2295
Cys Gly Asn Asn Ser Gly Gln Pro Ser Thr Val Val Asp Asn Thr
                755                 760                 765
CTC GCA CTC ATG ACA GCT TTT CTC TAC GCA TAT GCC CGG CTC ACT 2340
Leu Ala Leu Met Thr Ala Phe Leu Tyr Ala Tyr Ala Arg Leu Thr
                770                 775                 780
GGA GAT CAC GCC TTC GAA CTC ATG GAC GAA AAC TTC GTC TTC GTC 2385
Gly Asp His Ala Phe Glu Leu Met Asp Glu Asn Phe Val Phe Val
                785                 790                 795
TGC AAT GGT GAT GAC AAC AAG TTT TCC ATG TCA CCT AGC TTC ATG 2430
Cys Asn Gly Asp Asp Asn Lys Phe Ser Met Ser Pro Ser Phe Met
                800                 805                 810
GCG AAG TTT GGA TGC GAT TTC TCC CCA TTC CTC TCT GAG CTC GGG 2475
Ala Lys Phe Gly Cys Asp Phe Ser Pro Phe Leu Ser Glu Leu Gly
                815                 820                 825
CTA ACC TAC GAA TTT GAT GAA GCA ACT GAA GAT ATC TGC GAA AAC 2520
Leu Thr Tyr Glu Phe Asp Glu Ala Thr Glu Asp Ile Cys Glu Asn
                830                 835                 840
CCC TAC ATG TCC TTG ACG ATG GTC CGC ACC TCG TTT GGG ATT GGT 2565
Pro Tyr Met Ser Leu Thr Met Val Arg Thr Ser Phe Gly Ile Gly
                845                 850                 855
TTC TCT CTC TCC ATT GAG CGC ATC GTT GCA ATC TTG CAG TGG AGT 2610
Phe Ser Leu Ser Ile Glu Arg Ile Val Ala Ile Leu Gln Trp Ser
                860                 865                 870
CGC GCC GGT GGA GTC CTG CAT GCC TAT CTC TCT GGC ATT GCC GCT 2655
Arg Ala Gly Gly Val Leu His Ala Tyr Leu Ser Gly Ile Ala Ala
                875                 880                 885
CTT TTC GAA TCT TTC AAC ACA CCC AAA CTC TTC AAT CTC GTG CAC 2700
Leu Phe Glu Ser Phe Asn Thr Pro Lys Leu Phe Asn Leu Val His

                890                 895                 900
ACA TAT CTC CTT TGG CTC GTC ACA GAG CAC GAG GAG GAA CTC TTC 2745
Thr Tyr Leu Leu Trp Leu Val Thr Glu His Glu Glu Glu Leu Phe
                905                 910                 915
TCC ATG ATG GAA CTC AAA GAC ATG TTC ATG CCC TTG CCC ACT AGG 2790
Ser Met Met Glu Leu Lys Asp Met Phe Met Pro Leu Pro Thr Arg
                920                 925                 930
GAA CAG ATA GCC TTG CTG CAC TAC GTT GGA ACT GAG CCC ATC GTG 2835
Glu Gln Ile Ala Leu Leu His Tyr Val Gly Thr Glu Pro Ile Val
                935                 940                 945
GAG GAA ACA TTT CTG CAA GCA GGA CAC GAA GAA CCA GAC CCC ATC 2880
Glu Glu Thr Phe Leu Gln Ala Gly His Glu Glu Pro Asp Pro Ile
                950                 955                 960
GTT CCC CCA GTT CCT GAC ACC GAT CTA ACG AAC ATG GCT GCC GCA 2925
Val Pro Pro Val Pro Asp Thr Asp Leu Thr Asn Met Ala Ala Ala
                965                 970                 975
CCA CCC GAC AAC AGA AAG TCG CGC GCC GTC ATC CCC CGT GGG ACC 2970
Pro Pro Asp Asn Arg Lys Ser Arg Ala Val Ile Pro Arg Gly Thr
                980                 985                 990
AGT GAC TGG AGC CTG CCT GAA CCC AAA ATG CGA ACA CTG GGT TTC 3015
Ser Asp Trp Ser Leu Pro Glu Pro Lys Met Arg Thr Leu Gly Phe
                995                 1000                1005
AAG TCC AAG ATC AAC ATC GAA ACA CTT GCC AAT GTC CCC GAC GGG 3060
Lys Ser Lys Ile Asn Ile Glu Thr Leu Ala Asn Val Pro Asp Gly
                1010                1015                1020
TAT ATG AAC ACC TTT GCA TCC GTT GCA ACT GAA TCG CAA CGT AGG 3105
Tyr Met Asn Thr Phe Ala Ser Val Ala Thr Glu Ser Gln Arg Arg
                1025                1030                1035
AAA TGG GAG GAG GCC GCA CGT GGC GAT TTC GGC ATC ACT GAC GAT 3150

Lys Trp Glu Glu Ala Ala Arg Gly Asp Phe Gly Ile Thr Asp Asp
                1040                1045                1050
GAG AAA TGG GAG AAG TTG CTC ATT GCT GCT TGT ATA TAT TTC GCA 3195
Glu Lys Trp Glu Lys Leu Leu Ile Ala Ala Cys Ile Tyr Phe Ala
                1055                1060                1065
GAC AAT GGC ACC TCA CCG AAT TTC GAC GAG GAA CTC ACT ATG GAG 3240
Asp Asn Gly Thr Ser Pro Asn Phe Asp Glu Glu Leu Thr Met Glu
                1070                1075                1080
GTT AAT GGC GGT CTC AAC TCC ATC AAA GAG TAC CCC GTT CGC CCC 3285
Val Asn Gly Gly Leu Asn Ser Ile Lys Glu Tyr Pro Val Arg Pro
                1085                1090                1095
TTT GTC GTT AGA GCC AAA AAG ATT TCC ACG CTG CGC AGA ATC TTT 3330
Phe Val Val Arg Ala Lys Lys Ile Ser Thr Leu Arg Arg Ile Phe
                1100                1105                1110
CGC TGT TAC TCC ATC GAA ACG AAG CTC ATG TTC GTG AAG CTG AGG 3375
Arg Cys Tyr Ser Ile Glu Thr Lys Leu Met Phe Val Lys Leu Arg
                1115                1120                1125
CGC GTG CCA CAC TGG GCC ATA AAG CAC GGA TGC CTC GAT GAG ATC 3420
Arg Val Pro His Trp Ala Ile Lys His Gly Cys Leu Asp Glu Ile
                1130                1135                1140
GTC TTT GAT TTC ATG ATC CCC GAC CAG TTC ACC TCT AGG ACT GCG 3465
Val Phe Asp Phe Met Ile Pro Asp Gln Phe Thr Ser Arg Thr Ala
                1145                1150                1155
CTT GAA ACA CTG AAG CAG ACC AAA CTG GCT GCC ATT GGT GTT GGC 3510
Leu Glu Thr Leu Lys Gln Thr Lys Leu Ala Ala Ile Gly Val Gly
                1160                1165                1170
ACA AGC AAT TCT CTC CTC ACC TCC GAG CAG ACG AAC ATG AGG ACC 3555
Thr Ser Asn Ser Leu Leu Thr Ser Glu Gln Thr Asn Met Arg Thr
                1175                1180                1185

ACT GAA ACC CGA CGG CGC AAT GAC TAC GAT GGT CAC GAG GCG CTC 3600 T
hr Glu Thr Arg Arg Arg Asn Asp Tyr Asp Gly His Glu Ala Leu
1190 1195 1200
CTC CGG TAGATCTCTC ATGCACTTGC ATTTCCTTTT AAGCAATGCA 3646
Leu Arg
1202
TTTCAATACG CTTTTAATTA TATCAAGCGT CAAGAATTTC TCCCGCTTGC AGTTAATCCT 3706
TATAGGGTGG CACTTTGTGT TATGCAATGG AAAATTCAGT GACGCAATCA ACCATCATTG 3766
GTTGGTGACA ACGCAGTACT TTCTTTATTT CACATCAAGC GACCAGCACA TCGCCCGCTT 3826
ACAGCTAATC CTACCTGAGG GTGGCACTCT GTGTTATGTA ATGGAATGTG CAATCTCGCA 3886
ACCAACCGTC ATTGGTTGAT GGTGCGGCCA ACACCCGCAC TCTTGTATAC CGGAAAGGTT 3946

SEQ ID NO:3
SEQUENCE TYPE:Nucleotide
SEQUENCE LENGTH:20 base pairs
PROPERTIES:
TCAGGAAAAG ATGATCCAGA 20

SEQ ID NO:4
SEQUENCE TYPE:Nucleotide
SEQUENCE LENGTH:20 base pairs
PROPERTIES:
CAGTGCTCCG CGAAGAGGCC 20

SEQ ID NO:5
SEQUENCE TYPE:Nucleotide
SEQUENCE LENGTH:20 base pairs
PROPERTIES:
GCAGGACACG AAGAACCAGA 20

SEQ ID NO:6

SEQUENCE TYPE:Peptide

SEQUENCE LENGTH:10 amino acids

PROPERTIES:

Ser Gly Lys Asp Asp Pro Asp Pro Ile Val

                5                   10

SEQ ID NO:7

SEQUENCE TYPE:Peptide

SEQUENCE LENGTH:10 amino acids

PROPERTIES:

Ala Gly His Glu Glu Pro Asp Pro Ile Val

                5                   10

## Claims

1. A nucleotide sequence encoding a coat protein gene of barley mild mosaic virus which is selected from the group consisting of:
(a) a DNA sequence encoding amino acids 798 to 1048 of SEQ ID No. 1 or a part thereof;
(b) a DNA sequence encoding amino acids 952 to 1202 of SEQ ID No. 2 or a part thereof;
(c) a DNA sequence having a molecular weight of 480,000 ± 10,000 Dalton and having the restriction enzyme cleavage sites shown below 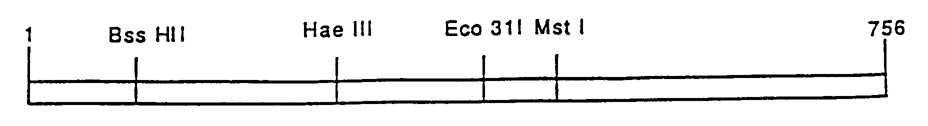
1 Bss HII Hae III Eco 31I Mst I 756
(d) a DNA sequence which is degenerate to a sequence according to any one of (a) to (b);
(e) a DNA sequence which is an allelic derivative of a sequence according to any one of (a) to (b);
(f) a nucleotide sequence hybridizing to a sequence according to any one of (a) to (e).

2. A recombinant DNA molecule containing a DNA sequence according to claim 1.

3. The recombinant DNA molecule according to claim 2 wherein said DNA sequence is under the control of regulatory elements allowing its expression in a desired host cell.
